# EUROPEAN PATENT SPECIFICATION

(11) **EP 0 904 071 B1**
(45) Date of publication and mention of the grant of the patent: **15.11.2000**
(21) Application number: 97920885.7
(22) Date of filing: 13.05.1997
(51) Int. Cl.: A61K 31/195

(54) **ORAL REHYDRATION PRODUCT COMPRISING GLUTAMINE**
GLUTAMINHALTIGES ORALES REHYDRATIONSPRODUKT
PRODUIT POUR REHYDRATATION ORALE CONTENANT DE LA GLUTAMINE

(30) Priority: 14.05.1996 GB 9609993
(43) Date of publication of application: 31.03.1999
(73) Proprietor: NORBROOK LABORATORIES LIMITED, Newry BT35 6JP (GB)
(72) Inventor: PATTERSON, Alan, Belfast BT4 2NQ (GB); ORR, Neil, Loughbrickland, Co. Down BT32 3RR (GB)
(74) Representative: Ede, Eric
(86) International application number: GB9701279
(87) International publication number: WO9742943

(56) References cited:
- WO-A-92/10175
- WO-A-93/25214
- JP-A- 6 209 719
- US-A- 5 288 703
- US-A- 5 561 111
- FOOD TECHNOLOGY, vol. 46, no. 4, - 1992 USA, pages 87-96, XP002039690 SCHMIDL M K AND LABUZA T P: "MEDICAL FOODS"

## Description

This invention relates to products for the treatment of mammals suffering from gut disorders which may arise from nutritional, bacterial, viral or protozoal causes and lead to fluid depletion, acidosis, and imbalances or loss of essential electrolytes. These problems typically arise in immature animals such as calves and lambs.

The intestinal losses of water and electrolytes, including bicarbonate, are major factors in the pathogenesis of diarrhoea (scour) in calves. Calf scour continues to represent a major financial loss to the farmer and also a great source of concern to the veterinary profession. This invention is primarily intended to be applied in the treatment of calves with diarrhoea and description of the invention herein will be related thereto. It will, however, be appreciated that other animals, including humans, may also suffer from abnormal conditions leading to similar dehydration, acidosis and electrolyte loss for which these products may be used.

The causes of dehydration have been well researched and although many interacting factors must be considered it is observed that severe dehydration includes reduced fluid absorption as well as increased secretion and is predisposed by loss of sodium and accompanied by imbalance of other electrolytes in the extracellular fluids. Acidosis, where blood pH drops from a normal range (7.34-7.40) to a lower range (perhaps 6.85-7.15) is mainly attributable to increases in loss of bicarbonate ions, increases in lactic acid (due to poor tissue perfusion and anaerobic metabolism) and additional factors such as increases in organic acid production by intestinal bacteria.

Losses of sodium and bicarbonate ions are considered to be the most serious aspects of diarrhoea but losses of potassium and calcium are also observed. It is therefore necessary to provide a satisfactorily formulated oral rehydration solution (ORS) for the treatment of diarrhoea, both in humans and animals.

There are many products currently available for use in non-human animals which have varying degrees of efficacy with respect to replacement of electrolytes, correction of acidosis and provision of an energy source. The Applicant's own Life Aid P (trade mark) is one such rehydration product, whilst another is Lectade Plus (trade mark of SmithKline Beecham Animal Health). Applicant's earlier invention in the same field is described in EP-A-O 644 767.

Whereas rehydration products may be administered intravenously, orally, or subcutaneously, the oral route is much preferred unless dehydration has already progressed to a serious level (8-10% or greater loss of bodyweight) when a combination of parenteral and oral administration should be employed. Conventional oral rehydration solutions are, however, formulated to address the dehydration, acidosis and electrolyte problems of diarrhoea but their use imposes virtual starvation because, in comparison to milk, the solutions are calorie deficient. Nutritional oral rehydration compositions aim to achieve energy levels more comparable with that of the natural milk denied to the animal during the use of an ORS.

An object of this invention is to provide an improved veterinary product providing effective rehydration whilst countering metabolic acidosis, calorific deficit and bodyweight loss more effectively than was previously possible, whilst also accelerating the recovery of enteric form and function.

A further object of this invention is to provide a product capable of supporting the structure and function of the intestine itself.

Accordingly this invention provides an oral rehydration composition, especially a nutritional oral rehydration composition, wherein the composition is one to be made up in water at point of use as an oral rehydration formulation, characterised in that said composition comprises glutamine, present in an amount effective to enhance nutritional uptake, an intimate mixture of metabolizable energy source, electrolytes, and bicarbonate precursors and physiologically acceptable cations including sodium. The concentration of sodium being about 120 millimoles per litre (mmol/l) of final formulation. The composition preferably also contains potassium chloride to obtain an ionically balanced product.

Preferably also the composition contains calcium chloride and magnesium acetate in order to provide free calcium and magnesium ions for ionic replacement.

Preferably the composition contains differing bicarbonate precursors to increase the probability of utilisation. The precursors are provided as a plurality of physiologically acceptable carboxylic acid anions with corresponding physiologically acceptable cations including sodium and other alkali metals, the yield of bicarbonate obtainable in use being about 80 mmol/l of final formulation. The most preferred group of precursors includes alkali metal, especially sodium, salts of a tricarboxylic acid and two other carboxylic acids. Propionate (propanoate), acetate and citrate are the preferred bicarbonate precursors being converted to this anion in vivo to yield the desired concentration. Metabolism of theseprecursors also contributes to the energy yield of the present product.

The composition of this invention also contains a significantly greater level of energy source than the current market leading prior art compositions, preferably about 380 mmol/l, and this is preferably as glucose which also enhances the uptake of sodium. Glutamine is included at a concentration of about 30 mmol/l to enhance the uptake of sodium ions, the absorption of glucose and as an additional energy source. The glutamine facilitates the metabolic utilisation of the absorbed glucose and also has beneficial effects on intestinal cells. The more effective use of glucose thus achieved avoids the need to consider even higher glucose content which could, at excessive concentration, draw water from the tissues into the gut and also provide a substrate for bacterial fermentation, both with detrimental effects.

The composition is preferably provided in particulate form, e.g. as a substantially dry powder or granules but may alternatively be provided as a liquid concentrate for dilution when required for administration.

In the form of an intimate mixture of dry (typically not more than about 1.8 % w/w water content) components of this invention (which may include a desiccant), the formulation is storage stable for extended periods.

The product may be produced for distribution as a substantially dry powder composition, or as a liquid concentrate for make up to a dosage formulation at point of use. Furthermore, this invention is suitable for packaging and supply as a single mix in a dosage quantity in a container such as a sachet or capsule, for example.

Preferably the unitary dosage package contains components to be made up in water at point of use as an oral rehydration formulation comprising an intimate mixture of Dextrose anhydrous 75.7%w/w, Sodium chloride 3.4%w/w, Potassium chloride 2.5%w/w, Sodium citrate dihydrate 5.4%w/w, Sodium acetate 0.7%w/w, Sodium propionate 1.1%w/w, Glutamine 4.9%w/w with the balance consisting of a dessicant and a colouring additive.

Advantageously the liquid concentrate when made up with water according to directions provided therewith yields a formulation in liquid form for oral administration comprising about 378.0 mmol/l Dextrose, about 30.0 mmol/l Glutamine, about 120.0 mmol/l Sodium, about 16.7 mmol/l Citrate, about 20 mmol/l Acetate, about 10.0 mmol/l Propionate, about 6.0 mmol/l Magnesium, about 9.0 mmol/l Calcium, about 100 mmol/l Chloride, and about 30.0 mmol/l Potassium.

The following examples illustrate the ionic strength of the invention when prepared for use.

**Table 1:**

| **Solution compositions** | **I (mmol/l)** | **II (mmol/l)** | **III (mmol/l)** | **IV (mmol/l)** |
|---|---|---|---|---|
| Sodium chloride | 52.00 | 52.00 | 52.00 | 52.00 |
| Potassium chloride | 30.00 | 30.00 | 30.00 | 30.00 |
| Sodium citrate | 16.70 | 16.70 | 16.70 | 16.70 |
| Sodium acetate | 8.00 | 8.00 | 8.00 | 8.00 |
| Sodium propionate | 10.00 | 10.00 | 10.00 | 10.00 |
| Calcium chloride | 9.00 | 9.00 | 9.00 | 9.00 |
| Magnesium acetate | 6.00 | 6.00 | 6.00 | 6.00 |
| Glucose | 100.00 | 378.00 | 378.00 | 756.00 |
| Glutamine | 5.00 | 30.00 | 45.00 | 60.00 |

The invention will now be further described by way of the following example. **(Example 1)**

A trial was conducted in diarrhoeic calves to ascertain the benefit from the inclusion of glutamine in veterinary ORS solutions. The trial was based on the methods published by Michell et al. (1992, Br. vet. J., 148, 505-522, Appendix 1) .

Three test solutions were used in the trial, B, G and Y. The formulations of the solutions used are shown in Table 2.

Solution B represents a control solution. Solution G tested the effect of adding calcium and magnesium ions as the concentrations of these ions often fall in diarrhoeic calves, especially during the period of administration of oral rehydration therapy. The inclusion of these divalent cations would not be expected to have any other function than ionic replacement. Solution Y was to test for beneficial effects of glutamine addition.

**Table 2:**

| **Solution compositions** | **B (mmol/l)** | **G (mmol/l)** | **Y (mmol/l)** |
|---|---|---|---|
| Sodium chloride | 40.00 | 52.00 | 52.00 |
| Potassium chloride | 20.00 | 30.00 | 30.00 |
| Sodium citrate | 16.70 | 16.70 | 16.70 |
| Sodium acetate | 20.00 | 8.00 | 8.00 |
| Sodium propionate | 10.00 | 10.00 | 10.00 |
| Calcium chloride | 0.00 | 9.00 | 9.00 |
| Magnesium acetate | 0.00 | 6.00 | 6.00 |
| Glucose | 378.00 | 378.00 | 378.00 |
| Glutamine | 0.00 | 0.00 | 30.00 |

The central tenet of oral rehydration therapy is that it minimises further fluid loss and helps replace fluid lost during the diarrhoeic process. Using the study design outlined below, two direct measurements of body fluid are feasible in a controlled study with full laboratory back up. The first of these is Plasma Volume, representative of the fluid in circulation in the bloodstream. The second is Extracellular Fluid which, in addition to plasma fluid, includes the fluid bathing cells in the animal's tissues. Blood volume can also be calculated from plasma volume and packed cell volume.

In the trial, after induction of diarrhoea, the calves received two litres of the relevant ORS twice daily for two days, i.e. four consecutive feeds. Thus, the data to the 48 hour timepoint represents the effect of administration of the pure ORS only. For the next four feeds, calves received two litres of 50% ORS/milk substitute solution (consistent with the recommended usage specified by most manufacturers), with the exception of some calves from Groups B and Y which received the ORS alone for four days, i.e. eight consecutive feeds. Given that there was no significant difference in the body fluid data between these two subgroups for both solution B and Y, the data are combined for analysis. In the trial, the calves which showed no decline in both ECF and Plasma Volume following the induction of diarrhoea, prior to the initiation of treatment, were excluded from the analysis of changes in ECF, Plasma Volume. The results of the trial are shown in Tables 3, 4, 5, 6 and 7.

**Table 3:**

| **Change in ECF Volume (litres) (± SE)** | | | | |
|---|---|---|---|---|
| **GROUP** | **Pre-diarrhoea** | **Pre-treatment (0 hrs)** | **Post-treatment (0-48 hrs)** | **Post-treatment (0-96 hrs)** |
| Group B | 15.5 | -0.5 | -2.1 | -1.5 |
| (n=12) | 0.7 | 0.3 | 0.5 | 0.3 |
| Group Y | 15.3 | -1.4 | -0.4 | -0.6 |
| (n=11) | 0.9 | 0.4 | 0.7 | 0.7 |
| Group G | 14.6 | -1.0 | -1.4 | -1.1 |
| (n=7) | 0.6 | 0.9 | 1.1 | 1.3 |

**Table 4:**

| **Change in Plasma Volume (litres) (± SE)** | | | | |
|---|---|---|---|---|
| **GROUP** | **Pre-diarrhoea** | **Pre-treatment (0 hrs)** | **Post-treatment (0-48 hrs)** | **Post-treatment (0-96 hrs)** |
| Group B | 3.1 | -0.4 | -0.1 | 0.1 |
| (n=12) | 0.1 | 0.1 | 0.1 | 0.1 |
| Group Y | 3.0 | -0.5 | 0.3 | 0.3 |
| (n=11) | 0.2 | 0.1 | 0.1 | 0.1 |
| Group G | 2.8 | -0.4 | 0.1 | 0.1 |
| (n=7) | 0.2 | 0.1 | 0.1 | 0.1 |

**Table 5:**

| **Bodyweight (kg) (± SE)** | | | |
|---|---|---|---|
| **GROUP** | **Pre-treatment (0 hrs)** | **Post-treatment (96 hrs)** | **Post-treatment (0-96 hrs change)** |
| Group B | 44.25 | 41.92 | -2.3 |
| (n=12) | 2.2 | 2.3 | 0.4 |
| Group Y | 42.0 | 41.5 | -0.5 |
| (n=10) | 1.0 | 1.0 | 0.4 |
| Group G | 41.79 | 39.21 | -2.6 |
| (n=7) | 2.4 | 1.9 | 0.9 |

**Table 6:**

| **Change in Blood Volume (litres) (± SE)** | | | | |
|---|---|---|---|---|
| **GROUP** | **Pre-diarrhoea** | **Pre-treatment (0 hrs)** | **Post-treatment (0-48 hrs)** | **Post-treatment (0-96 hrs)** |
| Group B | 5.2 | -0.5 | -0.1 | 0.0 |
| (n=12) | 0.3 | 0.2 | 0.2 | 0.1 |
| Group Y | 4.9 | -0.8 | 0.4 | 0.3 |
| (n=11) | 0.3 | 0.1 | 0.1 | 0.1 |
| Group G | 4.8 | -0.5 | 0.1 | 0.1 |
| (n=7) | 0.3 | 0.2 | 0.1 | 0.1 |

**Table 7:**

| **Change in PCV (ml/dl) (± SE)** | | | | |
|---|---|---|---|---|
| **GROUP** | **Pre-diarrhoea** | **Pre-treatment (0 hrs)** | **Post-treatment (0-48 hrs)** | **Post-treatment (0-96 hrs)** |
| Group B | 39.5 | 1.4 | -0.3 | -0.9 |
| (n=12) | 2.0 | 0.8 | 0.9 | 0.9 |
| Group Y | 38.0 | 1.5 | -1.5 | -1.6 |
| (n=11) | 1.9 | 1.5 | 1.3 | 1.0 |
| Group G | 40.7 | 2.1 | -0.6 | -2.0 |
| (n=7) | 2.9 | 1.6 | 1.5 | 1.8 |

Solution Y was the only one to significantly increase blood volume within 48 hours (p < 0.01) and sustain the increase subsequently. It was also the only one to correct the packed cell volume (PCV) within 48 hours and sustain the recovery subsequently (Table 6). These results are important because PCV, the proportion of blood occupied by red cells, is the main determinant of its viscosity; high viscosity seriously impedes capillary blood flow. Blood volume is a less direct measurement than plasma volume (from which it is derived, using PCV) but it is the determinant of circulatory 'fill'.

The nutritional benefits of glutamine addition can clearly be seen from the results, especially the body weight data for these calves over the study period. Whilst all three solutions contained the same amount of dextrose, as an energy source and to enhance sodium absorption, the group treated with solution Y demonstrated no statistically significant decrease in bodyweight over the period of treatment, whereas B and G did (p < 0.001, p < 0.05). The above formulations constitute a single therapy for the treatment of a calf suffering from scour and address the areas of electrolyte replacement, fluid replacement, correction of acidosis and provision of an energy source, provided the appropriate dose is given and repeated as specified.

### Example 2

A commercial product provided as a unitary dosage package to be made up at point of use as an oral rehydration formulation consists of an intimate mixture of Dextrose anhydrous 75.7%w/w, Sodium chloride 3.4%w/w, Potassium chloride 2.5%w/w, Sodium citrate dihydrate 5.4%w/w, Sodium acetate 0.7%w/w, Sodium propionate 1.1%w/w, Glutamine 4.9%w/w with the balance consisting of a dessicant and a colouring additive.

### Example 3

A similar product to be sold in liquid form to be prepared as a concentrate at a convenient volume which when maade up with water according to directions to be provided upon the package yields a formulation in liquid form for oral administration consisting of about 378.0 mmol/l Dextrose, about 30.0 mmol/l Glutamine, about 120.0 mmol/l sodium, about 16.7 mmol/l Citrate, about 20 mmol/l Acetate, about 10.0 mmol/l Propionate, about 6.0 mmol/l Magnesium, about 9.0 mmol/l Calcium, about 100 mmol/l Chloride, and about 30.0 mmol/l Potassium.

An advantage of the above-mentioned invention is that it can be used over a longer period at full strength compared with other ORS products. This is because the high calorie content in the product of the invention avoids virtual starvation, again unlike those of the traditional prior art. Furthermore, because most of the calories in the product are derived from glucose, additional beneficial effects of glutamine on glucose utilisation can be seen.

Because the formulation can be used for a longer period at full strength compared with other oral rehydration products, the glutamine can act for long enough to allow beneficial effects on gut cells (which are renewed in about three days). In conjunction with there being no detrimental effects on body fluid levels, as previously described, there was, in fact, a positive effect on plasma glucose levels and body weight by the prolonged administration of the pure ORS. Calves treated with solutions B and Y for four days showed a rise of 3.1 + 1.0 mmol/l in plasma glucose whereas those given 50% ORS/milk for the last four feeds had a rise of only 0.7 + 0.3 mmol/l. Similarly, those calves treated with solutions B and, Y for four days lost 0.5 + 0.5 kg, whereas those treated with 50% ORS/milk for the last four feeds lost 1.5 ± 0.5 kg bodyweight.

## Claims

1. An oral rehydration composition, wherein the composition is one to be made up in water at point of use as an oral rehydration formulation, characterised in that said composition comprises glutamine present in an amount effective to enhance nutrional uptake, an intimate mixture of metabolizable energy source, electrolytes, bicarbonate precursors, and physiologically acceptable cations including sodium.

2. An oral rehydration composition according to claim 1 wherein the composition also contains potassium chloride to obtain an ionically balanced product.

3. An oral rehydration composition according to claim 1 wherein the composition also contains calcium chloride and magnesium acetate in order to provide free calcium and magnesium ions for ionic replacement.

4. An oral rehydration composition according to claim 1 wherein the composition contains differing bicarbonate precursors to increase the probability of utilisation.

5. An oral rehydration composition according to claim 4 wherein the precursors are provided as a plurality of physiologically acceptable carboxylic acid anions with corresponding physiologically acceptable cations including sodium and other alkali metals.

6. An oral rehydration composition according to claim 5 wherein the precursors consist of alkali metal salts of a tricarboxylic acid and two other carboxylic acids.

7. An oral rehydration composition according to claim 6 wherein the preferred bicarbonate precursors are selected from the group consisting of propionates (propanoate), acetate and citrate, same being converted to this anion *in vivo* to obtain the desired bicarbonate yield.

8. An oral rehydration composition according to claim 1 wherein the yield of bicarbonate obtainable in use is about 80 mmol/l of final formulation.

9. An oral rehydration composition according to claim 1 wherein the composition contains a high level of energy source in the form of glucose.

10. An oral rehydration composition according to claim 9 wherein the amount of glucose in the composition is about 380 mmol/l.

11. An oral rehydration composition according to claim 1 wherein glutamine is included at a concentration of about 30 mmol/l.

12. An oral rehydration composition according to claim 1 wherein the composition is provided in particulate form, e.g. as a substantially dry powder or granules.

13. An oral rehydration composition according to claim 12 wherein the composition is in the form of a mixture of dry components of not more than 1.8% w/w water content.

14. An oral rehydration composition according to claim 12 wherein the dry components include a dessicant.

15. An oral rehydration composition according to claim 1 wherein the composition is provided as a liquid concentrate for dilution when required for administration.

16. An oral rehydration composition such as that claimed in claim 1 packaged such as to permit selection and delivery of a single dosage e.g. in single usage container such as a sachet or capsule.

17. A unitary dosage package of components to be made up in water at point of use as an oral rehydration formulation which comprises an intimate mixutre of dextrose anhydrous 75.7%w/w, sodium chloride 3.4%w/w, potassium chloride 2.5%w/w, sodium citrate dihydrate 5.4%w/w, sodium acetate 0.7%w/w, sodium propionate 1.1%w/w, glutamine 4.9%w/w with the balance consisting of a desiccant and a colouring additive.

18. A formulation in liquid form for oral administration comprising about 378.0 mmol/l dextrose, about 30.0 mmol/l glutamine, about 120.0 mmol/l sodium, about 16.7 mmol/l citrate, about 20 mmol/l acetate, about 10.0 mmol/l propionate, about 6.0 mmol/l magnesium, about 9.0 mmol/l calcium, about 100 mmol/l chloride, and about 30.0 mmol/l potassium.

## Patentansprüche

1. Eine orale Rehydrationszusammensetzung, wobei die Zusammensetzung eine solche ist, die am Verwendungsort als eine orale Rehydrationszusammensetzung gebildet wird, dadurch gekennzeichnet, daß die Zusammensetzung Glutamin in einer solchen Menge aufweist, die wirksam ist, um die Nährstoffaufnahme zu erhöhen, desweiteren eine innige Mischung aus metabolisierbarer Energiequelle, Elektrolyte, Bicarbonatvorläufer, und physiologisch annehmbaren Kationen, einschließlich Natrium.

2. Eine orale Rehydrationszusammensetzung nach Anspruch 1, wobei die Zusammensetzung auch Kalium-Chlorid enthält, um ein ionisch ausbalanciertes Produkt zu erhalten.

3. Eine orale Rehydrationszusammensetzung nach Anspruch 1, wobei die Zusammensetzung auch Calcium-Chlorid und Magnesium-Acetat enthält, um freie Calcium- und Magnesium-Ionen zum ionischen Ersatz zu liefern.

4. Eine orale Rehydrationszusammensetzung nach Anspruch 1, wobei die Zusammensetzung unterschiedliche Bicarbonatvorläufer enthält, um die Wahrscheinlichkeit der Verwendung zu erhöhen.

5. Eine orale Rehydrationszusammensetzung nach Anspruch 4, wobei die Vorläufer aus einer Mehrzahl von physiologisch annehmbaren Carboxyl-Säure-Anionen vorgesehen werden, mit entsprechenden physiologisch annehmbaren Kationen, einschließlich Natrium und anderen Alkalimetallen.

6. Eine orale Rehydrationszusammensetzung nach Anspruch 5, wobei die Vorläufer aus Alkalimetallsalzen einer tricarboxylischen Säure und zwei anderen carboxylischen Säuren bestehen.

7. Eine orale Rehydrationszusammensetzung nach Anspruch 6, wobei die vorgezogenen Bicarbonatvorläufer ausgewählt werden aus der Gruppe, die aus Propionaten (Propanoat), Acetat und Citrat besteht, wobei diese in vivo zu ihrem Anion umgesetzt werden, um die gewünschte Bicarbonatausbeute zu erhalten.

8. Eine orale Rehydrationszusammensetzung nach Anspruch 1, wobei die Ausbeute von Bicarbonat, die bei der Verwendung erreichbar ist, etwa 80 mmol/l der endgültigen Zusammensetzung beträgt.

9. Eine orale Rehydrationszusammensetzung nach Anspruch 1, wobei die Zusammensetzung ein hohes Maß von Energiequelle in der Form von Glucose enthält.

10. Eine orale Rehydrationszusammensetzung nach Anspruch 9, wobei die Menge von Glucose in der Zusammensetzung etwa 380 mmol/l beträgt.

11. Eine orale Rehydrationszusammensetzung nach Anspruch 1, wobei Glutamin mit einer Konzentration von etwa 30 mmol/l eingeschlossen ist.

12. Eine orale Rehydrationszusammensetzung nach Anspruch 1, wobei die Zusammensetzung in partikulierter Form vorgesehen ist, beispielsweise als ein im wesentlichen trocknes Pulver oder Granulat.

13. Eine orale Rehydrationszusammensetzung nach Anspruch 12, wobei die Zusammensetzung in der Form einer Mischung aus trocknen Komponenten vorliegt, die nicht mehr als 1,8 %w/w Wassergehalt aufweisen.

14. Eine orale Rehydrationszusammensetzung nach Anspruch 12, wobei die trocknen Komponenten ein Trocknungsmittel umfassen.

15. Eine orale Rehydrationszusammensetzung nach Anspruch 1, wobei die Zusammensetzung als ein flüssiges Konzentrat vorgesehen wird, das, wenn zur Zufuhr notwendig, verdünnt wird.

16. Eine orale Rehydrationszusammensetzung, wie die, die im Anspruch 1 beansprucht wird, derart verpackt, daß eine Auswahl und Ausgabe einer einzigen Dosis z.B. in einem Einmalbehälter, wie beispielsweise einen Beutel oder einer Kapsel, ermöglicht wird.

17. Eine unitäre Dosierungspackung von Komponenten, die am Ort der Verwendung als eine orale Rehydrationszusammensetzung in Wasser gebildet werden, welche eine innige Mischung aus 75,7 %w/w wasserfreier Dextrose, 3,4 %w/w Natrium-Chlorid, 2,5 %w/w Kalium-Chlorid, 5,4 %w/w Natrium-Citrat-Dihydrat, 0,7 %w/w Natrium-Acetat, 1,1 %w/w Natrium-Propionat, 4, 9 %w/w Glutamin, mit dem Rest bestehend aus Trocknungsmittel und einem färbendem Additiv, umfaßt.

18. Eine Zusammensetzung in flüssiger Form zur oralen Anwendung, umfassend etwa 378,0 mmol/l Dextrose, etwa 30,0 mmol/l Glutamin, etwa 120,0 mmol/l Natrium, etwa 16,7 mmol/l Citrat, etwa 20 mmol/l Acetat, etwa 10,0 mmol/l Propionat, etwa 6,0 mmol/l Magnesium, etwa 9,0 mmol/l Calcium, etwa 100 mmol/l Chlorid, und etwa 30,0 mmol/l Kalium.

## Revendications

1. Une composition pour réhydratation orale, laquelle est une composition à compléter dans de l'eau au point d'utilisation en tant que formulation de réhydratation, caractérisée en ce que ladite composition comprend de la glutamine présente en quantité efficace pour augmenter la prise de nutriments, un mélange intime de source d'énergie pouvant être métabolisée, d'électrolytes, de précurseurs de bicarbonate et de cations physiologiquement acceptables, dont du sodium.

2. Une composition pour réhydratation orale selon la revendication 1, laquelle composition contient également du chlorure de potassium pour obtenir un produit équilibré ioniquement.

3. Une composition pour réhydratation orale selon la revendication 1, laquelle composition contient également du chlorure de calcium et de l'acétate de magnésium afin que des ions calcium et magnésium libres soient disponibles pour le remplacement ionique.

4. Une composition pour réhydratation orale selon la revendication 1, laquelle composition contient des précurseurs de bicarbonate différents pour augmenter la probabilité d'utilisation.

5. Une composition pour réhydratation orale selon la revendication 4, dans laquelle les précurseurs sont prévus sous la forme d'une pluralité d'anions d'acide carboxylique physiologiquement acceptables avec des cations physiologiquement acceptables correspondants dont du sodium et d'autres métaux alcalins.

6. Une composition pour réhydratation orale selon la revendication 5, dans laquelle les précurseurs consistent en des sels métalliques alcalins d'un acide tricarboxylique et de deux autres acides carboxyliques.

7. Une composition pour réhydratation orale selon la revendication 6, dans laquelle les précurseurs de bicarbonate préférés sont sélectionnés dans le groupe comprenant les propionates (propanoate), acétates et citrates, ceux-ci étant convertis en cet anion in vivo pour obtenir le taux de bicarbonate désiré.

8. Une composition pour réhydratation orale selon la revendication 1, dans laquelle la quantité de bicarbonate pouvant être obtenue lors de l'utilisation est d'environ 80 mmol/l de la formulation finale.

9. Une composition pour réhydration orale selon la revendication 1, laquelle composition contient un taux élevé de source d'énergie sous la forme de glucose.

10. Une composition pour réhydration orale selon la revendication 9, dans laquelle la quantité de glucose dans la composition est d'environ 380 mmol/l.

11. Une composition pour réhydration orale selon la revendication 1, dans laquelle la glutamine est présente selon une concentration d'environ 30 mmol/l.

12. Une composition pour réhydration orale selon la revendication 1, laquelle composition est prévue sous une forme particulière, par exemple en tant que poudre ou granulés pratiquement secs.

13. Une composition pour réhydration orale selon la revendication 12, laquelle composition est sous la forme d'un mélange de composants secs ayant une teneur d'eau pas supérieure à 1,8 % en poids.

14. Une composition pour réhydration orale selon la revendication 12, dans laquelle les composants secs comprennent un déshydratant.

15. Une composition pour réhydration orale selon la revendication 1, laquelle composition est prévue sous la forme d'un liquide concentré à diluer lorsque nécessaire pour administration.

16. Une composition pour réhydration orale selon la revendication 1, conditionnée de manière à permettre la sélection et la fourniture d'une dose unique, par exemple dans un contenant à usage unique tel qu'un sachet ou une capsule.

17. Ensemble en contenant à dosage nique de composants à ajouter dans l'eau au point d'utilisation pour obtenir une formulation pour réhydratation orale, qui comprend un mélange intime de 75,7 % en poids de dextrose anhydre, de 3,4 % en poids de chlorure de sodium, de 2,5 % en poids de chlorure de potassium, de 5,4 % en poids de citrate de sodium dihydraté, de 0,7 % en poids d'acétate de sodium, de 1,1 % en poids de propionate de sodium, de 4,9 % en poids de gultamine, le complément consistant en un additif déshydratant et colorant.

18. Une formulation sous forme liquide pour administration orale comprenant environ 378,0 mmol/1 de dextrose, environ 30,0 mmol/l de glutamine, environ 120,0 mmol/l de sodium, environ 16,7 mmol/l de citrate, environ 20 mmol/l d'acétate, environ 10,0 mmol/1 de propionate, environ 6,0 mmol/l de magnésium, environ 9,0 mmol/l de calcium, environ 100 mmol/l de chlorure et environ 30,0 mmol/l de potassium.
